# EUROPÄISCHE PATENTSCHRIFT

(11) **EP 2 356 952 B1**
(45) Veröffentlichungstag und Bekanntmachung des Hinweises auf die Patenterteilung: **26.09.2012**
(21) Anmeldenummer: 11000565.9
(22) Anmeldetag: 25.01.2011
(51) Int. Cl.: A61B 19/02, A61L 2/26

(54) **Sterilbehälter**
Sterile container
Conteneur stérile

(30) Priorität: 26.01.2010 DE 202010001382 U
(43) Veröffentlichungstag der Anmeldung: 17.08.2011
(73) Patentinhaber: Gottfried Storz, Medizintechnik GmbH & Co. KG, 78532 Tuttlingen (DE)
(72) Erfinder: Kreidler, Winfried, 78532 Tuttlingen (DE)
(74) Vertreter: Neymeyer, Franz

(56) Entgegenhaltungen:
- DE-A1- 10 210 905
- DE-A1-102004 020 804
- DE-U1- 29 720 450
- DE-U1-202011 001 772

## Beschreibung

Die Erfindung betrifft einen Sterilbehälter bestehend aus einem kastenartigen Unterteil und einem Behälterdeckel, der zur Bildung eines geschlossenen Sterilraums abnehmbar und mittels umlaufend zwischen Behälterdeckel und Unterteil angeordneten Dichtungsmitteln luftdicht auf das Unterteil aufsetzbar und mit diesem verbindbar ist, wobei in einer Behälterwand Belüftungsöffnungen vorgesehen sind, durch welche ein Luftaustausch zwischen dem Innenraum und der Umgebung stattfindet, und wobei zwischen den Belüftungsöffnungen und dem Innenraum eine Sterilbarriere in Form einer Pasteurschen Schleife angeordnet ist, welche aus einer Sockelplatte und einer Deckelplatte besteht, die jeweils konzentrische Ringrippen aufweisen, welche mit radialem Spiel axial so ineinander greifen, dass sie axiale und radiale Ringspalte bilden und eine Sterilisation der durch die Belüftungsöffnungen eintretenden und diese Ringspalte abwechselnd radial und axial durchströmenden und schließlich in den Sterilraum gelangenden Luft bewirken.

Aus DE 10 2004 020 804 A1 (Basis für den Oberbegriff des Anspruchs 1) ist ein Sterilbehälter zur Aufnahme und zur Aufbewahrung von chirurgischem Besteck oder chirurgischem Material bekannt, der einen Aufnahmeraum aufweist, welcher durch einen Behälterboden und durch Behälterwände gebildet und durch einen Deckel verschließbar ist. Eine Sterilbarriere definiert dauerhaft einen Sterilströmungspfad zum Herstellen einer Fluidverbindung zwischen dem Aufnahmeraum und der Umgebung des Sterilbehälters. Außerdem ist bei diesem bekannten Sterilbehälter ein Überdruckströmungspfad vorgesehen, der ebenfalls eine Fluidverbindung zwischen dem Aufnahmeraum und der Umgebung definiert.

Dieser Überdruckströmungspfad ist jedoch geschlossen, wenn sich der Sterilbehälter in einer Sterilstellung befindet, in welcher ein Gasaustausch zwischen dem Aufnahmeraum und der Umgebung nur durch den Sterilströmungspfad möglich ist. Der Überdruckströmungspfad ist hingegen teilweise geöffnet, wenn sich der Sterilbehälter in einer Überdruckstellung befindet, in welcher eine Druckdifferenz zwischen den im Aufnahmeraum und in der Umgebung herrschenden Drücken eine Mindestdruckdifferenz übersteigt. Um dabei einen einfachen Aufbau und eine einfache Wartung zu ermöglichen, ist vorgesehen, dass ein Gasdurchtrittsquerschnitt des Sterilströmungspfades zum Ausbilden eines Überdrückströmungspfades veränderbar ist.

Der Sterilströmungspfad wird aus Ringvorsprüngen (Ringrippen) zweier Trägerplatten gebildet. Diese Ringvorsprünge der beiden Trägerplatten greifen derart wechselseitig ineinander, dass eine Sterilbarriere in Form eines mäanderförmigen Sterilströmungspfades mit der Wirkung einer Pasteurschen Schleife gebildet wird. Die Veränderbarkeit des Sterilströmungspfades wird dadurch realisiert, dass eine der Trägerplatten relativ zur anderen verstellbar ist.

Ein weiterer Sterilbehälter mit einer Pasteurschen Schleife als Sterilbarriere ist aus der DE 20 2006 003 591 U1 bekannt. Auch bei diesem Sterilbehälter ist in einem ersten Strömungspfad, der einen Medienaustausch während des Sterilisierungsvorganges ermöglicht, ein druckabhängig selbstschließendes Einlassventil angeordnet. Dieses Einlassventil öffnet beim Überschreiten einer vorbestimmten Druckdifferenz zwischen Außen- und Innendruck den ersten Strömungspfad nach dem Behälterinnern. Weiter ist in diesem Strömungspfad ein selbstschließendes Auslassventil vorgesehen, welches beim Überschreiten einer vorbestimmten Druckdifferenz zwischen Innen-und Außendruck den ersten Strömungspfad nach außen öffnet.

Sterilbehälter der gattungsgemäßen Art werden in Kliniken und Arztpraxen dazu benutzt, chirurgische Instrumente und medizinische Behandlungsmittel nach Gebrauch zu sterilisieren, d.h. von Krankheitskeimen zu befreien. Zu diesem Zweck werden die Instrumente und sonstigen Behandlungsmittel in einen solchen Sterilbehälter gelegt, der dann in einem Sterilisierungsschrank einer Heißluft-und/oder Dampfatmosphäre ausgesetzt wird. Diese Heißluft- und/oder Dampfatmosphäre dringt durch die vorgesehenen Belüftungsöffnungen in den Sterilraum ein, um die Sterilisation der darin befindlichen Einlagen zu bewirken. Danach soll zwar außerhalb des Sterilisierungsschrankes ein Luftaustausch zwischen dem Sterilraum und der Umgebung stattfinden, allerdings über eine Sterilbarriere, die das Eindringen von Krankheitskeimen bzw. -erregern in den Sterilraum verhindert.

Solche Sterilbarrieren können auch aus austauschbaren Papier- oder Textilfiltern bestehen, welche zum Abdecken der Belüftungsöffnungen benutzt werden. Auch werden als Sterilbarriere sogenannte Pasteursche Schleifen eingesetzt, die von zwei mit konzentrischen Ringrippen und dazwischen liegenden Ringspalten versehenen Platten gebildet sind. Diese Platten sind so zusammengefügt, dass ihre Ringrippen und Ringspalte verzahnungsartig, jedoch mit radialem Spiel ineinander greifen. Dabei werden radiale Durchlassspalte gebildet, durch welche eine von den Ringrippen mehrfach rechtwinklig umgelenkte Gas- oder Luftaustauschströmung entsteht, welche ihre eigene Sterilisierung bewirkt. Unterschiedliche Strömungspfade und druckabhängige Ventile, die nach jedem Reinigungsvorgang neu justiert werden müssen, verteuern die Herstellung, erschweren die Handhabung und beeinträchtigen die Funktionssicherheit.

Der Erfindung liegt die Aufgabe zugrunde die Sterilbarriere eines Sterilbehälters der eingangs genannten Art so zu gestalten, dass diese sich mit einer verbesserten Effektivität einfach und kostengünstig herstellen, montieren und zum Zwecke der Reinigung demontieren, d.h. handhaben lässt.

Gelöst wird diese Aufgabe erfindungsgemäß zusammen mit den Merkmalen des Oberbegriffes des Anspruches 1 dadurch, dass die konzentrischen Ringrippen der Sockelplatte und der Deckelplatte unterschiedliche axiale Höhen aufweisen, also hohe und niedere Ringrippen vorgesehen sind und, dass jeweils zwischen zwei hohen Ringrippen eine niedere Ringrippe angeordnet ist und, dass die niederen Ringrippen der Sockelplatte auf dem gleichen Radius liegen wie die hohen Ringrippen der Deckelplatte und, dass die einander jeweils paarweise zugeordneten niedrigen und hohen Ringrippen der Sockelplatte und der Deckelplatte einen umlaufend axialen, radial durchlässigen Ringspalt bilden.

Durch die Erfindungsgemäße Ausgestaltung wird eine erhebliche Verbesserung der Sterilisationswirkung der Pasteurschen Schleife erreicht. Durch die unterschiedlichen Höhen der einander zugeordneten Ringrippen der Sockelplatt und der Deckelplatte ergibt eine zusätzlich Umlenkung des Luftstromes innerhalb der labyrinthartigen Pasteurschen Schleife bzw. des "Labyrinthfilters", was die Sterilisationswirkung erhöht. Auch können dadurch die Strömungsquerschnitte so gestaltet werden, dass der Luft- bzw. Gasaustausch in der gewünschten Schnelligkeit erfolgen kann. Es ist auch gewährleistet, dass sich beim Zusammenbau der Deckelplatte und der Sockelplatte ohne besondere Aufmerksamkeit des Personals jeweils die gleichen Einstellungen ergeben. Somit kann auch erreicht werden, dass immer die gleichen Betriebsverhältnisse herrschen.

Durch die Ausgestaltung gemäß Anspruch 2 sind die Deckelplatte und die Sockelplatte sehr einfach zur Bildung der Sterilbarriere zusammenzufügen und zum Zweck der Reinigung auch leicht wieder voneinander trennbar. Eine solche Verbindung lässt sich leicht und sicher herstellen und auch wieder lösen. Die Handhabung wird insbesondere für ungeschultes Personal erleichtert. Dabei ist auch wichtig, die Verbindung zwischen der Sockelplatte und der Deckelwand oder dem Boden des Sterilbehälters möglichst so zu gestalten, dass diese einfach und kostengünstig herstellbar ist und sich ebenso leicht handhaben lässt. Deshalb sind die hier vorgesehenen Gewindeverbindungen von erheblichem Vorteil.

Um sicherzustellen, dass durch die Gewindeverbindung kein zweiter Luftaustauschpfad einstehen kann, ist gemäß Anspruch 3 das das Gegengewinde zum Gewindezapfen aufnehmende Innengewinde in einer Sackbohrung angeordnet.

Weil es erfahrungsgemäß schwierig ist, zwei große ebene Metallflächen luftdicht aufeinander zu pressen, ist gemäß Anspruch 4 die Sockelplatte auf ihrer der innenseitigen Anlegefläche des Behälterdeckels zugewandten Seite mit einer planebenen Ringfläche versehen, die sich ggf. mit Hilfe eines geeigneten Dichtungsmittels, z.B. eines Dichtungsrings, dicht auf die Innenfläche des Behälterdeckels aufsetzen und mit der Gewindeverbindung anpressen lässt.

Die Ausgestaltung nach Anspruch 5 ermöglicht die Herstellung der Verbindung zwischen der Sterilbarriere und der Umgebung auf einfache und sichere Weise.

Mit der Ausgestaltung nach Anspruch 6 werden die Belüftungsöffnungen des Behälterdeckels vor dem ungewollten Eindringen von Flüssigkeit, insbesondere von Spritzwasser und anderer Flüssigkeiten, die versehentlich auf die Außenfläche des Behälterdeckels gelangen können, geschützt.

Die Ausgestaltung nach Anspruch 7 ist insofern von Vorteil, als mit dem vorgesehenen Randring und der Gewindeverbindung leichter und sicherer ein flüssigkeitsdichtes Anliegen der Schutzkappe an der Außenfläche des Behälterdeckels erzielt werden kann.

Mit der Ausgestaltung nach Anspruch 8 ist zugleich sichergestellt, dass Flüssigkeit, die sich auf der Außenfläche des Behälterdeckels befindet, nicht durch die für den Luftaustausch vorgesehenen Durchlassöffnungen in das Innere der Schutzkappe und zu den Belüftungsöffnungen des Behälterdeckels gelangen kann, weil der Randring im Bereich der schlitzartigen Ausnehmungen, die gemäß Anspruch 6 von der Außenfläche des Behälterdeckels einen Mindestabstand aufweisen, eine Schwelle bzw. Sperre bildet.

Die Ausgestaltung nach Anspruch 9 gewährleistet einen ausreichend großen Einlassquerschnitt für den Luftaustausch.

Durch die Ausgestaltung nach Anspruch 10 ergibt sich eine sehr einfache Befestigungsmöglichkeit für die Sockelplatte am Behälterdeckel, wobei die weiteren Ausgestaltungen nach Anspruch 11 und 12 weitere Vereinfachungen und Kosteneinsparungen ermöglichen.

Mit der gemäß Anspruch 13 vorgesehenen Ringrippe an der Sockelplatte, die in eine passende Ringnut des Behälterdeckels eingreift, lässt sich nicht nur eine konzentrische Anordnung der Sockelplatte erreichen, sondern auch besser und einfacher eine dichte Verbindung zwischen Sockelplatte und Behälterdeckel, zumal sich so eine Ringrippe gemäß Anspruch 14 leicht mit einem Dichtungsmittel, wie z.B. mit einem elastischen Dichtungsring ausstatten lässt.

Zur einfachen und sicheren Abdichtung kann gemäß Anspruch 15 der Randring und die Ringnut mittels eines Dichtungsmittels, insbesondere mittels eines elastischen O-Ringes oder einer Silikondichtung, gegeneinander abgedichtet sein.

Anhand der Zeichnungen wird die Erfindung im Folgenden näher erläutert. Es zeigt:
- Fig. 1: eine Schnittdarstellung eines aus einem Unterteil und einem Behälterdeckel bestehenden Sterilbehälters;
- Fig. 2: einen Schnitt II - II des Unterteils aus Fig. 1;
- Fig. 3: den Behälterdeckel des Sterilbehälters aus Fig. 1 im Schnitt;
- Fig. 4: eine Unteransicht des Behälterdeckels aus Fig. 3 mit einstückig eingearbeiteter Sockelplatte einer in Fig. 7 im Schnitt dargestellten Pasteurschen Schleife;
- Fig. 5: einen Schnitt V-V des Behälterdeckels aus Fig. 4;
- Fig. 5a: eine vergrößerte Darstellung der rechten Hälfte der Sockelplatte aus Fig. 5;
- Fig. 6: eine zur Pasteurschen Schleife der Fig. 7 gehörige Deckelplatte im Schnitt;
- Fig. 6a: in vergrößerter Darstellung die rechte Hälfte der Deckelplatte aus Fig. 6;
- Fig. 7: eine Schnittdarstellung des Behälterdeckels einer durch Zusammenfügen der Sockelplatte des Behälterdeckels aus Fig. 4 und der Deckelplatte aus Fig. 6 komplettierten Pasteursche Schleife;
- Fig. 7a: in vergrößerter Darstellung die rechte Hälfte der Pasteurschen Schleife aus Fig. 7;
- Fig. 8: einen vergrößert dargestellten Ausschnitt VIII aus Fig. 7;
- Fig. 9: einen Schnitt IX-IX aus Fig. 8;
- Fig. 10: eine Draufsicht X aus Fig. 11 einer als separates Fertigungsteil hergestellten Sockelplatte einer Pasteurschen Schleife;
- Fig. 11: eine Schnittdarstellung XI-XI der Sockelplatte aus Fig. 10;
- Fig. 12: in isometrischer Unteransicht eine Schutzkappe mit zentralem Gewindezapfen;
- Fig. 13: die Schutzkappe der Fig. 12 im Schnitt XII-XII;
- Fig. 14: im Schnitt die Wand des Behälterdeckels, an deren Innenbzw. Unterseite die aus der Sockelplatte der Fig. 11 und der Deckelplatte der Fig. 15 gebildete Pasteursche Schleife befestigbar ist;
- Fig. 15: im Schnitt die zur Sockelplatte der Fig. 11 gehörige Deckelplatte;
- Fig. 16: die aus der Sockelplatte der Fig. 11 und der Deckelplatte der Fig. 15 gebildete Pasteursche Schleife;
- Fig. 17: eine Einzelheit aus Fig. 10 im Schnitt XVII-XVII;
- Fig. 18: die Deckelwand des Behälterdeckels mit einer anderen Sockelpatte;
- Fig. 19: im Schnitt eine zur Sockelplatte der Fig. 18 passende Deckelplatte;
- Fig. 20: im Schnitt die aus der Schutzkappe der Fig. 12, 13 und der Sockelplatte der Fig. 18 und der Deckelplatte der Fig. 19 gebildete Pasteursche Schleife;
- Fig. 21: im Schnitt die Pasteursche Schleife in einer Ausführung, bei der die Deckelplatte mit der Sockelplatte rastend in Eingriff steht;
- Fig. 22: eine Unteransicht der Fig. 21;
- Fig. 23: in vergrößerter Darstellung eine Rastvorrichtung als Einzelheit XXII aus Fig. 21.

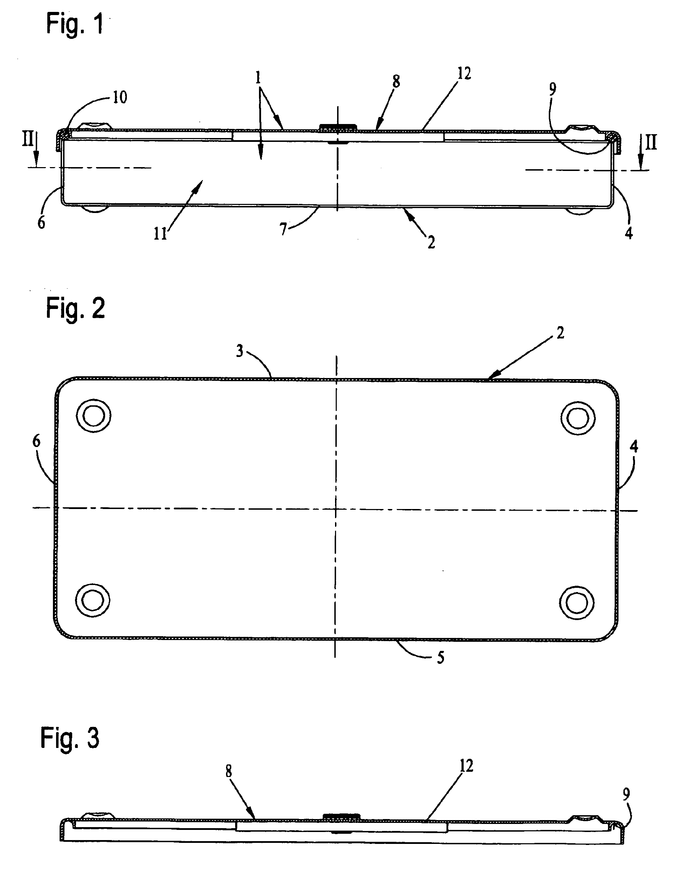

Der in den Fig. 1 und 2 dargestellte Sterilbehälter 1 besteht aus einem wannenartigen Unterteil 2 mit vier vertikalen Seitenwänden 3, 4, 5, 6 und einem Boden 7 aus Aluminium-Blech mit einer Dicke von etwa 1,5 bis 3 mm, die alle einstückig miteinander verbunden sind. Es handelt sich bei diesem Ausführungsbeispiel um einen kleinen Sterilbehälter von geringer Bauhöhe, der aus dem "Vollen" durch spanabhebende Bearbeitung einstückig hergestellt ist. Dicht verschließbar ist dieser Sterilbehälter 1 mit einem vorzugsweise aus demselben Material bestehenden Behälterdeckel 8.

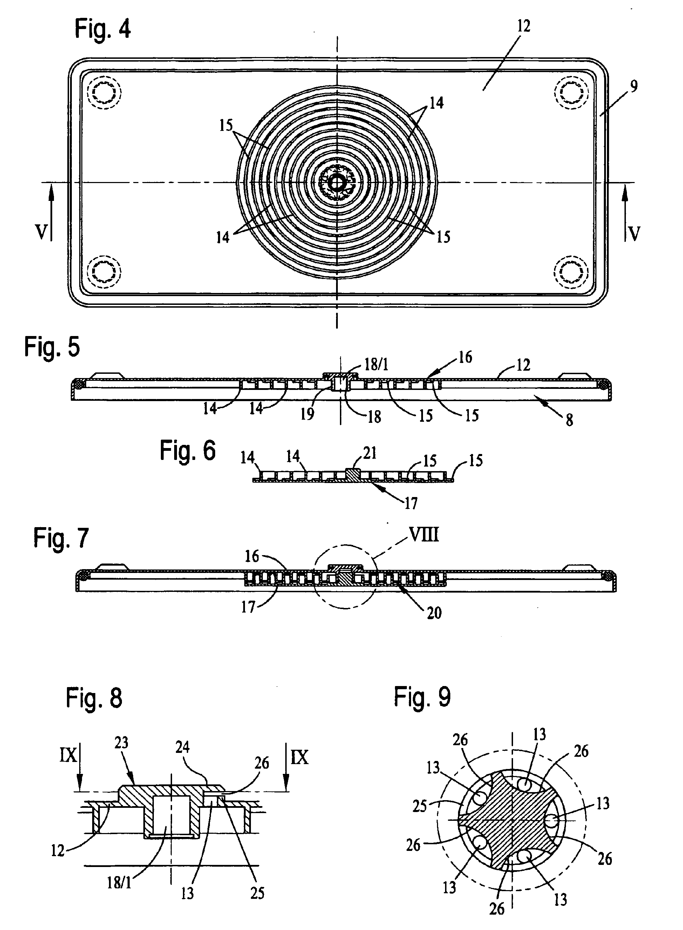

Dieser Behälterdeckel 8 ist mit einer innen umlaufenden Randrille 9 versehen, in welche ein elastischer Dichtungsring 10 eingelegt, vorzugsweise eingeklebt ist, der dichtend auf den in einer gemeinsamen Horizontalebene liegenden oberen Randkanten der Seitenwände 3 bis 6 aufliegt und ggf. mittels eines Spannverschlusses angepresst wird. Auch der Behälterdeckel 8 ist auf diese Weise einstückig hergestellt. Um zwischen dem Innenraum 11 des Sterilbehälters 1 und seiner Umgebung einen Luft- oder Gasaustausch zu ermöglichen, ist die Deckelwand 12 des Behälterdeckels 8 in ihrem Flächenzentrum mit einer Anzahl von aus zylindrischen Bohrungen bestehenden Belüftungsöffnungen 13 (Fig. 8 und 9) versehen.

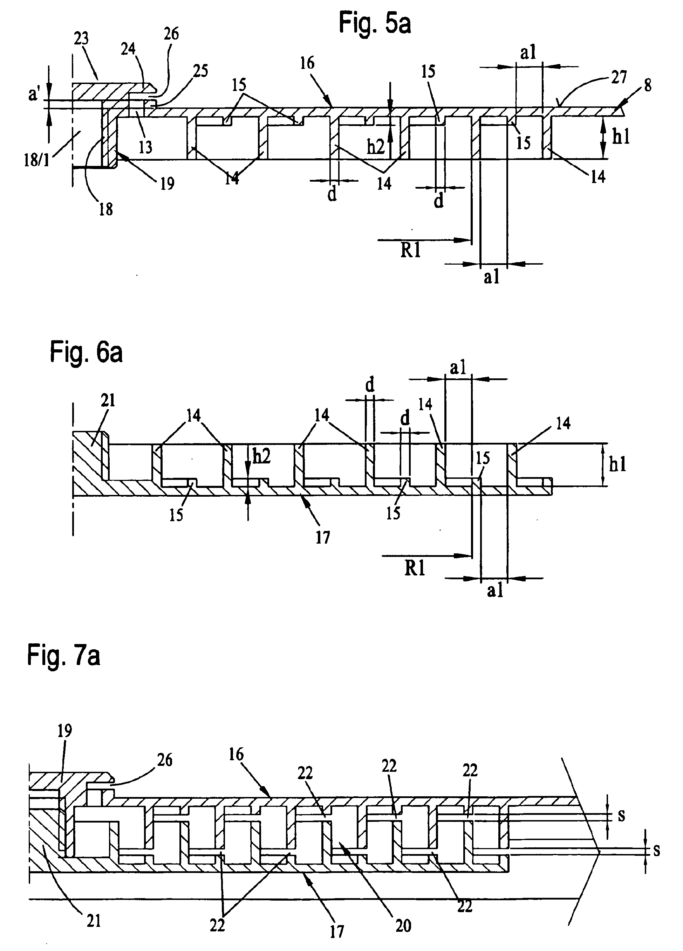

Wie am besten aus den Fig. 4, 5 und 5a ersichtlich ist, befinden sich auf der Innenseite der Deckelwand 12 des Behälterdeckels 8 mehrere zueinander konzentrische Ringrippen 14 und 15, die unterschiedliche axiale Höhen h1 und h2 aufweisen,(Fig. 5a). Dabei verläuft jeweils in der radialen Mitte zwischen zwei hohen Ringrippen 14 der Höhe **h1** eine niedrigere Ringrippe 15 mit der Höhe **h2**, die zusammen mit den Ringrippen 14 und 15 einer Deckelplatte 17 (Fig. 6, 6a) eine Pasteursche Schleife 20 bilden (Fig. 7 und 7a). Wie aus den Fig. 5a und 6a ersichtlich ist, haben die niedrigen Ringrippen 15 von den benachbarten hohen Ringrippen 14 jeweils den gleichen Abstand **a1.** Diese Ringrippen 14 und 15 sind einstückiger Bestandteil der Deckelwand 12 und bilden mit dieser Deckelwand 12 die Sockelplatte 16 der Pasteurschen Schleife 20 der Fig. 7, die hierbei als Sterilbarriere verwendet wird. Die dazugehörige Deckelplatte 17 ist in den Fig. 6 und 6a dargestellt. Auch diese Deckelplatte 17 ist mit mehreren zueinander konzentrischen Ringrippen 14 und 15 mit den unterschiedlichen axialen Höhen **h1** und **h2** versehen, wobei die niedrigen Ringrippen 15 mit der geringeren Höhe **h2** der Deckelplatte 17 jeweils auf dem Radius **R1** einer Ringrippe 14 der größeren Höhe **h1** der anderen Platte, d.h. der Sockelplatte 16 liegen.

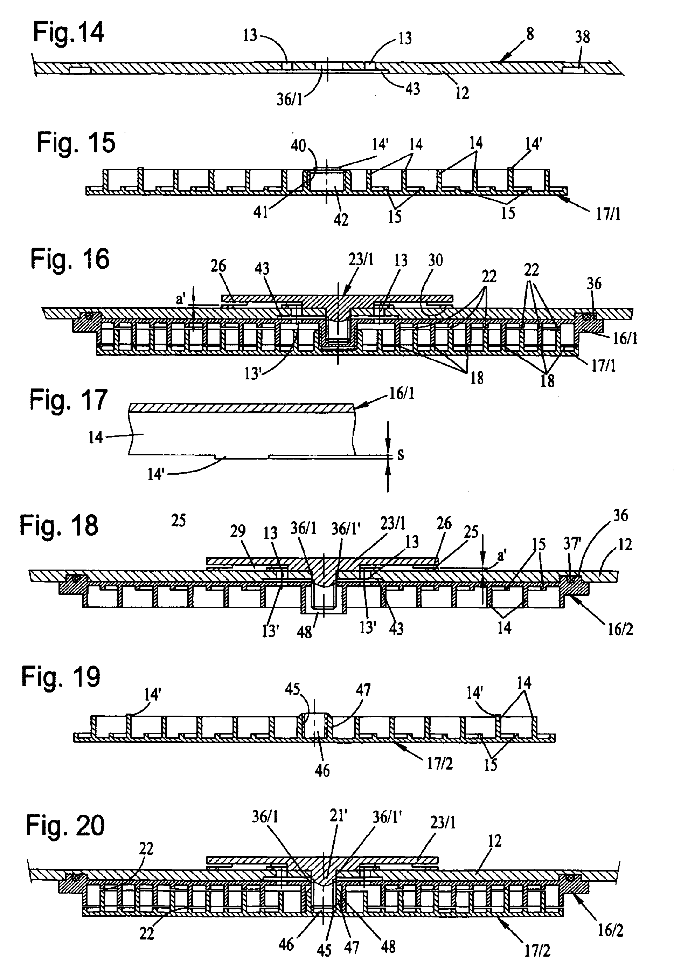

Auf diese Weise bilden die niedrigen Ringrippen 15 der einen Platte (Deckelplatte) 17 mit den hohen Ringrippen 14 der jeweils anderen Platte (Sockelplatte) 16 einen umlaufenden axialen Ringspalt 22 mit der Spaltbreite **s**. Diese Spaltbreite **s** wird bestimmt von der axialen Höhe mehrerer erhöhter Abschnitte 14' einer hohen Ringrippe 14 beispielsweise der Deckelplatte 17. Siehe hierzu insbesondere die Fig. 17.

Um die Deckelplatte 17 auf einfache Weise, also leicht handhabbar, mit der Sockelplatte 16 verbinden zu können, ist die Deckelplatte 17 im Zentrum der Ringrippen 14, 15 mit einer ein Innengewinde 18 aufweisenden Hohlnabe 19 versehen und die Sockelplatte 16 mit einem Gewindezapfen 21, der in diese Hohlnabe 19 eingeschraubt werden kann. Durch die konzentrische Anordnung der Ringrippen 14, 15 der Sockelplatte 16 zur Hohlnabe 19 bzw. der Deckelplatte 17 zum Gewindezapfen 21 ist sichergestellt, dass jeweils eine niedrige Ringrippe 15 der Sockelplatte 16 mit einer hohen Ringrippe 14 der Deckelplatte 17 fluchtet und so die Pasteursche Schleife 20 gebildet wird. Durch das Vorsehen der unterschiedlich hohen Ringrippen 14 und 15 an den beiden Sockelplatte 16 und der Deckelplatte 17 wird die Sterilisierwirkung der gebildeten Pasteurschen Schleife stark erhöht bzw. effektiver gestaltet, weil dadurch mehr und schärfere Strömungsumlenkungen innerhalb der Pasteurschen Schleife stattfinden. Dadurch kann eine Verringerung der Anzahl der Ringrippen 14, 15 ermöglicht werden, was zu einer Senkung der Herstellungskosten genutzt werden kann. Durch die erhöhten Abschnitte 14' an einer radial möglichst weit außen liegenden hohen Ringrippe 14 der Sockelplatte 16 oder der Deckelplatte 17 ist auch gewährleistet, dass bei ordnungsgemäßem Zusammenschrauben der beiden Platten 16 und 17 immer die gleichen Arbeits- bzw. Strömungsverhältnisse herrschen.

Das Innengewinde 18 der Hohlnabe 19 befindet sich in einer Sackbohrung 18/1 (Fig. 8), die keine Verbindung zur Umgebung zulässt. Dadurch ist zugleich sichergestellt, dass durch die Gewindeverbindung kein zweiter unkontrollierter Luftaustauschpfad entstehen kann.

Um die Belüftungsöffnungen 13 des Behälterdeckels 8 bzw. der Deckelwand 12 gegen das Eindringen von verschütteten Flüssigkeiten, und Spritzwasser zu schützen, ist auf der Außenseite der Deckelwand 12 des Behälterdeckels 8 eine scheibenartige Schutzkappe 23 ausgebildet (Fig. 8). Diese Schutzkappe 23 weist eine runde, flache Deckscheibe 24 auf, welche die Belüftungsöffnungen 13 der Deckelwand 12 oberseitig abdeckt. Zudem ist die Deckscheibe 24 mit einem Randring 25 versehen, der dicht auf der Außenfläche der Deckelwand 12 des Behälterdeckels 8 aufliegt oder im Falle der einstückigen Herstellung mit dieser spaltfrei verbunden ist (Fig. 9) .

Zur Herstellung einer Luftverbindung zwischen den Belüftungsöffnungen 13 und der Umgebung sind in den Randring 25 mehrere schlitzartige Durchbrüche 26 eingearbeitet, die jedoch von der Außenfläche 27 der Deckelwand 12 einen Abstand **a**' (Fig. 5a) aufweisen, so dass der Randring 25 gegenüber fließenden oder kriechenden Flüssigkeiten, die sich auf dem Behälterdeckel 8 befinden, ein unüberwindbare Hindernis bildet. Die zweckmäßigerweise mit einem Scheibenfräser von außen radial in die Deckscheibe 24 eingearbeiteten Durchbrüche 26 überschneiden sich mit den Belüftungsöffnungen 13, so dass diese Belüftungsöffnungen 13 mit der Umgebung in direkter Verbindung stehen (siehe Fig. 8 und 9).

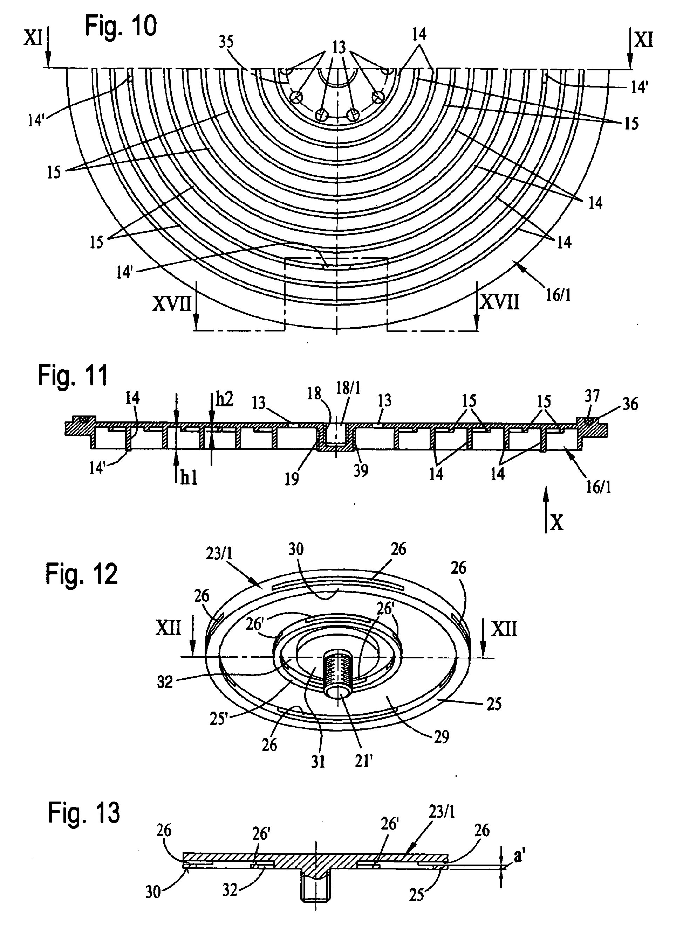

Bei der Ausführungsform der Fig. 10 bis 16 sind sowohl die Sockelplatte 16/1 als auch die Deckelplatte 17/1 und auch die Schutzkappe 23/1 jeweils als separate Fertigungsteile ausgebildet und mittels einer Gewindeverbindung an der Deckelwand 12 des Behälterdeckels 8 befestigt. Die in Fig. 10 hälftig in Unteransicht und in Fig. 11 im Schnitt dargestellte Sockelplatte 16/1 weist auf ihrer nach innen (unten) gerichteten Kreisfläche wie die Sockelplatte 16 mehrere konzentrische Ringrippen 14 und 15 mit unterschiedlichen axialen Höhen **h1** und **h2** auf, deren Ausbildung und Anordnung prinzipiell derjenigen entspricht, die in Verbindung mit den Fig. 5 bis 7a bereits beschrieben sind. Die Sockelplatte 16/1 der Fig. 10 und 11 kann allerdings einen größeren Durchmesser und damit auch mehr Ringrippen 14, 15 aufweisen als die Sockelplatte 16. Entsprechend ist auch dazu passend die zugehörige Deckelplatte 17/1 der Fig. 15 ausgebildet.

Die hierbei verwendete scheibenartige Schutzkappe 23/1 der Fig. 12, 13 und 16 weist einen umlaufenden Randring 25 auf, der einen flachen Hohlraum 29 umschließt und mit einer planebenen Auflagefläche 30 dicht auf der oberseitigen Fläche der Deckelwand 12 des Behälterdeckels 8 aufliegt. Auch der Randring 25 ist mit mehreren schlitzartigen Durchbrüchen 26 versehen, welche den Hohlraum 29 mit der Umgebung verbinden. Wie die Durchbrüche 26 der Schutzkappe 23, haben auch die Durchbrüche 26 der Schutzkappe 23/1 von der unteren Auflagefläche 30 des Randringes 25 bzw. von der oberseitigen Außenfläche des Behälterdeckels 8 einen Mindestabstand **a',** so dass auch hier der mit seiner Auflagefläche 30 auf dem Behälterdeckel 8 dicht aufliegende Randring 25 gegenüber fließenden oder kriechenden Flüssigkeiten, die sich auf dem Behälterdeckel 8 befinden, eine unüberwindbare Hemmschwelle bildet.

Im Zentrum eines unterseitigen runden Verstärkungssockels 31, der von einem inneren Ring 25' umgeben ist, weist die Schutzkappe 23/1 einen Gewindezapfen 21 auf. Zwischen dem Ring 25' und dem Verstärkungssockel 31 besteht ein ringförmiger Hohlraum 32, in den die Belüftungsöffnungen 13 der Deckelwand 12 münden und der über Schlitze 26' des Ringes 25' mit dem Hohlraum 29 in Verbindung steht, so dass die Belüftungsöffnungen 13 für einen Luftaustausch auch mit der Umgebung in Verbindung stehen. Die Belüftungsöffnungen 13 sind verteilt auf einem konzentrischen Kreis 35 angeordnet (Fig. 10), so dass sie etwa in der radialen Mitte des Hohlraums 32 der Schutzkappe 23/1 liegen. Beim Zusammenfügen der Bauteile wird der Gewindezapfen 21' durch eine zentrale Bohrung 36/1 (Fig. 14) der Deckelwand 12 des Behälterdeckels 8 geführt und in ein als Gegengewinde ausgebildetes Innengewinde 18 einer Sackbohrung 18/1 einer Hohlnabe 19 der Sockelplatte 16/1 geschraubt. Auf diese Weise wird die unterseitig mit den unterschiedlich hohen Ringrippen versehene Sockelplatte 16/1 an der Deckelwand 12 des Behälterdeckels 8 befestigt.

Um dabei eine möglichst dichte Verbindung zwischen der Deckelwand 12 und der Sockelplatte 16/1 erreichen zu können, ist die Sockelplatte 16/1 oberseitig mit einem erhabenen Randring 36 versehen, der mit einer dazu passenden unterseitigen Ringnut 38 der Deckelwand 12 in Eingriff gebracht wird.

Dabei können zusätzliche Dichtungsmittel, z. B. ein Dichtungsring 37' (siehe auch Fig. 16, 17) und/oder pastenartige oder kittförmige Dichtungsmittel Anwendung finden. Da diese Verbindung normalerweise dauerhaft sein und bei Reinigungsarbeiten nicht getrennt werden soll, lässt sich mit den heute zur Verfügung stehenden Dichtungstechniken bzw. Dichtungsmitteln eine dauerhaft dichte Verbindung zwischen der Sockelplatte 16/1 und der Deckelwand 12 realisieren. Wichtig ist dabei aber auch, dass die Gewindeverbindung zwischen Schutzkappe 23/1 und Sockelplatte 16/1 keinen unkontrollierten Gas- bzw. Luftaustausch zulässt. Deshalb ist das als Gegengewinde für den Gewindezapfen 21' vorgesehene Innengewinde 18 der Sockelplatte 16/1 in einer Sackbohrung 18/1 der Hohlnabe 19 angeordnet. Um die Deckelplatte 17/1 ebenfalls einfach und leicht mit der Sockelplatte 16/1 verbinden zu können, ist auch zwischen diesen beiden Bauteilen eine Gewindeverbindung vorgesehen. Diese besteht aus einen Außengewinde 39 der Hohlnabe 19 (Fig. 11) der Sockelplatte 16/1 und aus einem Innengewinde 40 einer Hohlnabe 41 (Fig. 15) der Deckelplatte 17/1, wobei auch dieses Innengewinde 40 in einer Sackbohrung 42 untergebracht ist.

Damit beim Zusammenschrauben der Teile nicht darauf geachtet werden muss, dass die Belüftungsöffnungen 13 der Deckelwand 12 mit den Belüftungsbohrungen 13' der Sockelplatte 16/1 fluchten (Fig. 18), ist an der Unterseite der Deckelwand 12 in dem Bereich, in dem sich die Belüftungsöffnungen 13 und die Belüftungsbohrungen 13' befinden, eine einen Verbindungshohlraum bildende Ausnehmung 43 angebracht.

Bei der in den Fig. 18, 19 und 20 dargestellten Ausführungsform ist der Gewindezapfen 21' der Schutzkappe 23/1 ebenfalls durch eine zentrale Bohrung 36/1 der Deckelwand 12 und durch eine dazu koaxiale Bohrung 36/1' in der Sockelplatte 16/2 geführt und in ein Innengewinde 45 einer Sackbohrung 46 einer zentralen Hohlnabe 47 der Deckelplatte 17/2 eingeschraubt. Dabei ist die Deckelplatte 17/2 mit einer zentralen, die Bohrung 36/1' konzentrisch umschließenden buchsenartigen Nabe 48 versehen, in welche die mit dem Innengewinde 45 versehene Hohlnabe 47 der Deckelplatte 17/2 eintaucht, wenn die Teile zusammengeschraubt werden, wie in Fig. 20 dargestellt. Auch hierbei ist durch erhöhte Abschnitte 14', wie auch in Fig. 17 beispielhaft für die Sockelplatte 16/1 dargestellt, auf einer oder mehreren hohen Ringrippen 14 der Sockelplatte 16/2 oder der Deckelplatte 17/2 sichergestellt, dass die Ringspalte 22 entstehen.

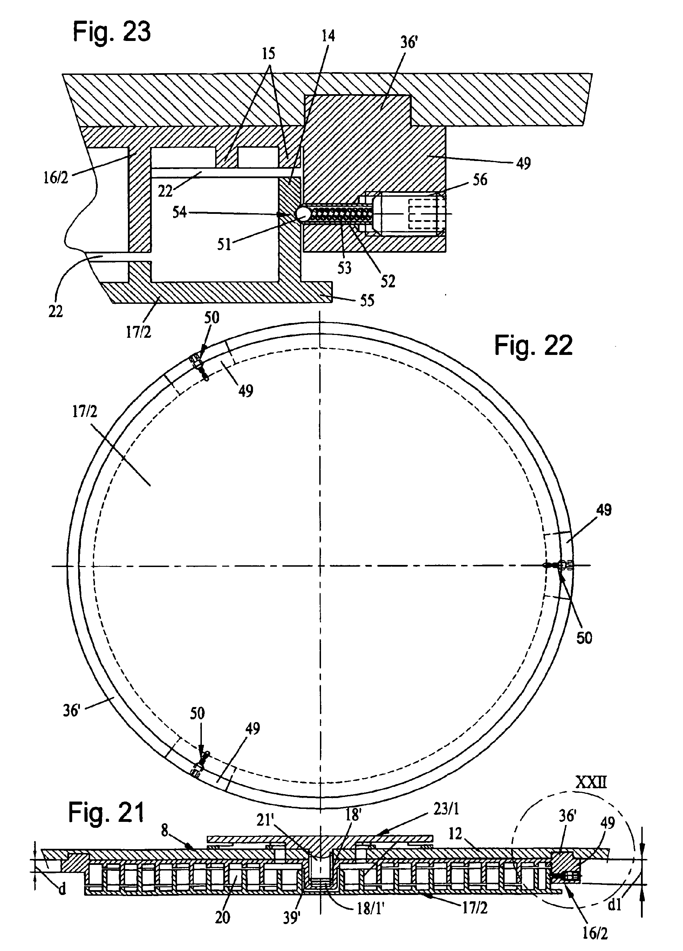

In Fig. 21 ist eine der Fig. 16 entsprechende Pasteursche Schleife 20 dargestellt, bei der die aus einem separaten Fertigungsteil bestehende Sockelplatte 16/2 mittels eines Gewindezapfens 21' einer Schutzkappe 23/1 innenseitig an einer Deckelwand 12 eines Behälterdeckels 8 befestigt ist. Dabei ist der einstückig mit der Schutzkappe 23/1 verbundene Gewindezapfen 21' in ein Innengewinde 18' (siehe auch Bezugszeichen 18 in Fig. 11) eingeschraubt, das sich in einer Sackbohrung 18/1' einer Hohlnabe 39' befindet. Mit einer solch festen Verbindung zwischen Deckelwand 12 und Sockelplatte 16/2 ist es ohne Weiteres möglich, zur Befestigung der Deckelplatte 17/2 an der Sockelplatte 16/2 eine leicht handhabbare Rastverbindung vorzusehen, damit die Deckelplatte 17/2 leicht und einfach auf die Sockelplatte 16/2 aufgesetzt bzw. von dieser gelöst werden kann.

Eine solche Möglichkeit ist bei der in den Fig. 21 bis 23 dargestellten Ausführungsform verwirklicht. Dazu ist der Randring 36' der Sockelplatte 16/2 an drei um jeweils 120° zueinander in Umfangsrichtung versetzten Stellen mit radial und axial von einem kleineren Maß **d** auf das größere Maß **d1** verdickten Abschnitten 49 versehen, in denen jeweils Rasteinrichtungen 50 angeordnet sind.

Wie insbesondere aus Fig. 23 ersichtlich ist, bestehen diese Rasteinrichtungen 50 jeweils aus einer Rastkugel 51, die im Endbereich eines mit einem Gewindekopf versehenen, am Ende eingebördelten Führungsrohres 52 gegen die Wirkung einer Druckfeder 53 axial beweglich gelagert ist. Die Rastkugel 51 greift zugleich rastend in eine umlaufende Rastkerbe 54 der äußeren Ringrippe 14 der Deckelplatte 17/2 ein. Um beim Einsetzen der Deckelplatte 17/2 in die Sockelplatte 16/2 ein sicheres Einrasten der Rastkugel 51 in die Rastkerbe 54 zu gewährleisten, ist die Sockelplatte 16/2 mit einer der Ringrippe 14 der Deckelplatte 17/2 gegenüberstehenden niedrigen Ringrippe 15 versehen. Zudem kann die hohe Ringrippe 14 der Deckelplatte 17/2 oder der Sockelplatte 16/2 stirnseitig mit mehreren erhöhten Abschnitten 14'(siehe Fig. 17 für die Sockelplatte 16/1) versehen sein, welche die Ringspalte 22 erzeugen (Fig. 23).

An dem radial überstehenden Rand 55 kann die Deckelplatte 17/2 zum Zwecke des Abziehens mit den Fingern einer Hand leicht und sicher erfasst werden. Bei dieser Ausführungsform entfällt die Gewindeverbindung zwischen der Sockelplatte 16/2 und der Deckelplatte 17/2.

## Patentansprüche

1. Sterilbehälter (1) bestehend aus einem kastenartigen Unterteil (2) und einem Behälterdeckel (8), der zur Bildung eines geschlossenen Sterilraums (11) abnehmbar und mittels umlaufend zwischen Behälterdeckel (8) und Unterteil (2) angeordneten Dichtungsmitteln luftdicht auf das Unterteil (2) aufsetzbar und mit diesem verbindbar ist, wobei in einer Behälterwand (12) Belüftungsöffnungen (13) vorgesehen sind, durch welche ein Luftaustausch zwischen dem Innenraum (11) und der Umgebung stattfindet, und wobei zwischen den Belüftungsöffnungen (13) und dem Innenraum (11) eine Sterilbarriere in Form einer Pasteurschen Schleife (20) angeordnet ist, welche aus einer Sockelplatte (16) und einer Deckelplatte (17) besteht, die jeweils konzentrische Ringrippen (14, 15) aufweisen, welche mit radialem Spiel axial so ineinander greifen, dass sie axiale und radiale Ringspalte (22) bilden und eine Sterilisation der durch die Belüftungsöffnungen (13) eintretenden und diese Ringspalte (22) abwechselnd radial und axial durchströmenden und schließlich in den Sterilraum (11) gelangenden Luft bewirken,
**dadurch gekennzeichnet,**
**dass** die konzentrischen Ringrippen (14, 15) der Sockelplatte (16) und der Deckelplatte (17) unterschiedliche axiale Höhen (h1, h2) aufweisen, also hohe und niedere Ringrippen vorgesehen sind und,
**dass** jeweils zwischen zwei hohen Ringrippen (14) eine niedere Ringrippe (15) angeordnet ist und,
**dass** die niederen Ringrippen (15) der Sockelplatte (16) auf dem gleichen Radius (R1) liegen wie die hohen Ringrippen (14) der Deckelplatte (17) und,
**dass** die einander jeweils paarweise zugeordneten niedrigen und hohen Ringrippen (15 und 14) der Sockelplatte (16) und der Deckelplatte (17) einen umlaufend axialen, radial durchlässigen Ringspalt (22) bilden.

2. Sterilbehälter nach Anspruchs 1, **dadurch gekennzeichnet, dass** die Deckelplatte (17) mit der an der Innenseite des Behälterdeckels (8) oder des Behälterbodens (7) einstückig mit diesem ausgebildeten oder als separates Fertigungsteil an dessen Innenseite angeordneten und mittels einer Gewindeverbindung oder einer Renkverbindung daran befestigten Sockelplatte (16) mittels wenigstens einer federnden Schnapp- oder Rastverbindung (50) lösbar in Eingriff steht.

3. Sterilbehälter nach Anspruch 2, **dadurch gekennzeichnet, dass** die mittels einer Gewindeverbindung mit dem Behälterdeckel (8) oder mit dem Gehäuseboden (7) verbundene Sockelplatte (16) als Gegengewinde zu einem Gewindezapfen (21') ein Innengewinde (18) in einer Sackbohrung (18/1) einer zentralen Hohlnabe (19) aufweist.

4. Sterilbehälter nach Anspruch 1 oder 2, **dadurch gekennzeichnet, dass** die Sockelplatte (16) in wenigstens einem radial und/oder axial verdickten Umfangsabschnitt (49) ihrer radial äußeren Ringrippe oder eines Randringes (36') mit einem radial federnd ausgebildeten Rastelement (50) versehen ist, das kraftschlüssig rastend in eine Rastrille (54) od. dgl. einer Ringrippe (14) der Deckelplatte (17) eingreift.

5. Sterilbehälter nach Anspruch 1 oder 2, **dadurch gekennzeichnet, dass** die an der Innenseite des Behälterdeckels (8) als separates Fertigungsteil angeordnete Sockelplatte (16) wenigstens mit einer sich auf der den Ringrippen (14, 15) abgewandten Planseite befindenden, planebenen Ringfläche an einer ebenen Anlagefläche der Innenseite des Behälterdeckels (8) dicht anliegt.

6. Sterilbehälter nach Anspruch 2, **dadurch gekennzeichnet, dass** die Sockelplatte (16) in einem zwischen der innersten Ringrippe und dem Gewinde liegenden Radialbereich mit Verbindungsöffnungen (13') zu den ebenfalls in diesem Radialbereich angeordneten Belüftungsöffnungen (13) des Behälterdeckels (8) versehen ist.

7. Sterilbehälter nach Anspruch 1 oder 2, **dadurch gekennzeichnet, dass** auf der Außenseite des Behälterdeckels (8) eine scheibenförmige Schutzkappe (23) ausgebildet oder aufgesetzt ist, welche die Belüftungsöffnungen des Deckels mit axialem Abstand abdeckt und welche in einem umlaufenden Randring (25) mit wenigsten einer radialen Durchlassöffnung (26) versehen ist, welche von der den Randring (25) aufnehmenden Außenfläche (27) des Behälterdeckels (8) einen axialen Mindestabstand (a') aufweist.

8. Sterilbehälter nach Anspruch 7, **dadurch gekennzeichnet, dass** ein Randring (25) einer auf der Außenseite des Behälterdeckels (8) aufgesetzten Schutzkappe (23) einen flachen, mit den Belüftungsöffnungen (13) des Deckels (8) in Verbindung stehenden Hohlraum (29) umschließt und mit einer planebenen Ringfläche (30) an der Außenfläche (27) des Behälterdeckels (8) dicht anliegt.

9. Sterilbehälter nach Anspruch 7, **dadurch gekennzeichnet, dass** die radiale Durchlassöffnung des Randrings (25) aus einer schlitzartigen, parallel zu seiner planebenen Ringfläche verlaufenden Ausnehmung (26) besteht.

10. Sterilbehälter nach Anspruch 8 oder 9, **dadurch gekennzeichnet, dass** der Randring (25) mit mehreren in Umfangsrichtung verteilt angeordneten schlitzartigen Durchlassöffnungen (26) versehen ist.

11. Sterilbehälter nach Anspruch 3 oder 4, **dadurch gekennzeichnet, dass** die Sockelplatte (16/1) mittels eines eine Bohrung (36/1) des Behälterdeckels (8) durchragenden Gewindezapfens (21), der mit einem zentralen Gegengewinde (18) der Sockelplatte (16/1) oder der Deckelplatte (17/1) in Eingriff steht, am Behälterdeckel (8) lösbar befestigt ist.

12. Sterilbehälter nach Ansprüche 3 und 9, **dadurch gekennzeichnet, dass** mit dem Gewindezapfen (21') auch die Schutzkappe (23) auf der Außenseite des Behälterdeckels (8) befestigt ist.

13. Sterilbehälter nach Anspruch 12, **dadurch gekennzeichnet, dass** der Gewindezapfen (21') einstückiger Bestandteil der Schutzkappe (23) ist.

14. Sterilbehälter nach Anspruch 1, 2 oder 3, **dadurch gekennzeichnet, dass** die Sockelplatte (16/1) mittels eines in eine passende, zum Ringbereich der Belüftungsöffnungen (13) konzentrische Ringnut (38) des Behälterdeckels (8) eingreifenden Randringes (36) zentriert ist.

15. Sterilbehälter nach Anspruch 14, **dadurch gekennzeichnet, dass** der Randring (36) und die Ringnut (38) mittels eines Dichtungsmittels, insbesondere mittels eines elastischen O-Ringes oder einer Silikondichtung, gegeneinander abgedichtet sind.

## Claims

1. Sterile container (1) consisting of a box-like lower part (2) and a container lid (8), which for the formation of a closed sterile chamber (11) can be removed and by means of sealing means arranged peripherally between the container lid (8) and the lower part (2) can be fitted in an air-tight manner onto the lower part and connected to the latter, wherein ventilation openings (13) are provided in a container wall (12), through which an exchange of air occurs between the inner chamber (11) and the environment, and wherein between the ventilation openings (13) and the inner chamber (11) a sterile barrier is provided in the form of a Pasteur loop (20), which consists of a base plate (16) and a cover plate (17) each comprising concentric annular ribs (14, 15) which engage axially with one another with radial play so that they form axial and radial annular gaps (22) and perform the sterilisation of the air entering through the ventilation openings (13) and flowing through said annular gaps (22) radially and axially alternately and finally reaching the sterile chamber (11),
**characterised in that**
the concentric annular ribs (14, 15) of the base plate (16) and the cover plate (17) have different axial heights (h1, h2), thus high and low annular ribs are provided and,
**in that** between two high annular ribs (14) a low annular rib (15) is arranged and,
**in that** the low annular ribs (15) of the base plate (16) lie on the same radius (R1) as the high annular ribs (14) of the cover plate (17) and
**in that** the low and high annular ribs (15 and 14) of the base plate (16) and the cover plate (17) assigned to one another in pairs form an axially circumferential, radially permeable annular gap (22).

2. Sterile container according to claim 1, **characterised in that** the cover plate (17) is releasably connected to the base plate (16) formed on the inner side of the container lid (8) or the container base (7) in one piece with the latter or arranged as a separate production part on its inner side and secured thereto by means of a threaded connection or a bayonet connection by means of at least one elastic snap-in or locking connection (50).

3. Sterile container according to claim 2, **characterised in that** the base plate (16) connected by means of a threaded connection to the container lid (8) or the housing base (7) comprises an internal thread (18) in a blind bore (18/1) of a central hollow hub (19) as a counter thread to a threaded pin (21').

4. Sterile container according to claim 1 or 2, **characterised in that** the base plate (16) is provided in at least one radially and/or axially thickened circumferential section (49) of its radially outer annular rib or edge ring (36') with a radially resilient locking element (50), which engages in a force-fitting manner in a locking groove (54) or the like of an annular rib (14) of the cover plate (17).

5. Sterile container according to claim 1 or 2, **characterised in that** the base plate (16) arranged on the inner side of the container lid (8) as a separate production part fits tightly with at least with one planar annular surface located on the plane side facing away from the annual ribs (14, 15) against a plane bearing surface of the inner side of the container lid (8).

6. Sterile container according to claim 2, **characterised in that** the base plate (16) is provided in a radial area lying between the innermost annular rib and the thread with connection openings (13') to the ventilation openings (13) of the container lid (8) also arranged in this radial area.

7. Sterile container according to claim 1 or 2, **characterised in that** on the outside of the container lid (8) a disc-like protective cap (23) is formed or fitted, which covers the ventilation openings of the lid with axial spacing and which is provided in a circumferential edge ring (25) with at least one radial through opening (26), which has an axial minimum distance (a') from the outer surface (27) of the container lid (8) mounting the edge ring (25).

8. Sterile container according to claim 7, **characterised in that** an edge ring (25) of a protective cap (23) fitted on the outside of the container lid (8) surrounds a flat hollow chamber (29) in connection with the ventilation openings (13) of the lid (8) and with a planar annular surface (30) fits tightly on the outer surface (27) of the container lid (8).

9. Sterile container according to claim 7, **characterised in that** the radial through opening of the edge ring (25) consists of a slot-like recess (26) running parallel to its planar annular surface.

10. Sterile container according to claim 8 or 9, **characterised in that** the edge ring (25) is provided with a plurality of slot-like through openings (26) distributed in circumferential direction.

11. Sterile container according to claim 3 or 4, **characterised in that** the base plate (16/1) is secured detachably to the container lid (8) by means of a threaded pin (21) projecting through a bore (36/1) of the container lid (8) which pin engages with a central counter thread (18) of the base plate (16/1) or the cover plate (17/1).

12. Sterile container according to claims 3 and 9, **characterised in that** by means of the threaded pin (21') also the protective cap (23) is secured onto the outside of the container lid (8).

13. Sterile container according to claim 12, **characterised in that** the threaded pin (21') is a one-piece component of the protective cap (23).

14. Sterile container according to claim 1, 2, or 3, **characterised in that** the base plate (16/1) is centred by means of an edge ring (36) which engages in a fitting annular groove (38) of the container lid (8) concentric to the annular area of the ventilation openings (13).

15. Sterile container according to claim 14, **characterised in that** the edge ring (36) and the annular groove (38) are sealed from one another by means of a sealing device, in particular by means of an elastic O-ring or a silicon seal.

## Revendications

1. Conteneur stérile (1) comprenant une partie inférieure (2) de type caisson et un couvercle (8) qui, en vue de former un espace stérile confiné (11), peut être mis amoviblement en place sur ladite partie inférieure (2), avec étanchéité à l'air assurée par des moyens d'étanchement périphériquement interposés entre ledit couvercle (8) du conteneur et ladite partie inférieure (2), à laquelle il peut être relié, une paroi (12) dudit conteneur étant percée d'orifices de ventilation (13) par l'intermédiaire desquels un échange d'air s'opère entre l'espace intérieur (11) et l'espace environnant, sachant qu'une barrière stérile revêtant la forme d'une boucle de Pasteur (20), interposée entre lesdits orifices de ventilation (13) et ledit espace intérieur (11), est composée d'une platine d'embase (16) et d'une platine de recouvrement (17) respectivement pourvues de nervures annulaires concentriques (14, 15) s'interpénétrant dans le sens axial, avec jeu radial, de manière à former des interstices annulaires (22) axiaux et radiaux, et à provoquer une stérilisation de l'air qui afflue en empruntant lesdits orifices de ventilation (13), circule radialement et axialement en alternance par ces interstices annulaires (22) et parvient, pour finir, dans ledit espace stérile (11),
**caractérisé par le fait**
**que** les nervures annulaires concentriques (14, 15) de la platine d'embase (16) et de la platine de recouvrement (17) présentent des hauteurs axiales différentes (h1, h2), c'est-à-dire que des nervures annulaires hautes et basses sont prévues,
**qu'**une nervure annulaire basse (15) est respectivement interposée entre deux nervures annulaires hautes (14),
**que** les nervures annulaires basses (15) de la platine d'embase (16) se situent sur le même rayon (R1) que les nervures annulaires hautes (14) de la platine de recouvrement (17), et que les nervures annulaires basses et hautes (15 et 14) de ladite platine d'embase (16) et de ladite platine de recouvrement (17), associées les unes aux autres par paires respectives, forment un interstice annulaire (22) qui s'étend axialement à la périphérie et est perméable dans le sens radial.

2. Conteneur stérile selon la revendication 1, **caractérisé par le fait que** la platine de recouvrement (17) est en prise libérable, à l'aide d'au moins une liaison élastique (50) par déclic ou par crantage, avec la platine d'embase (16) qui est réalisée d'un seul tenant avec la face intérieure du couvercle (8) du conteneur ou du fond (7) dudit conteneur, ou est disposée à la face intérieure de ce dernier, en tant que pièce distincte, et est fixée à ce dernier au moyen d'une liaison filetée ou d'une liaison à baïonnette.

3. Conteneur stérile selon la revendication 2, **caractérisé par le fait que** la platine d'embase (16), reliée au couvercle (8) du conteneur ou au fond (7) dudit conteneur au moyen d'une liaison filetée, comporte un filetage intérieur (18) qui constitue un filetage complémentaire d'un tenon fileté (21'), et est pratiqué dans un trou borgne (18/1) d'un moyeu central creux (19).

4. Conteneur stérile selon la revendication 1 ou 2, **caractérisé par le fait que** la platine d'embase (16) est dotée, sur au moins un tronçon périphérique (49) à renflement radial et/ou axial de sa nervure annulaire située radialement à l'extérieur, ou d'un anneau marginal (36'), d'un élément encliquetable (50) doué d'élasticité radiale et pénétrant par crantage, avec engagement positif, dans une rainure d'encliquetage (54) ou une configuration similaire d'une nervure annulaire (14) de la platine de recouvrement (17).

5. Conteneur stérile selon la revendication 1 ou 2, **caractérisé par le fait que** la platine d'embase (16), disposée à la face intérieure du couvercle (8) dudit conteneur en tant que pièce distincte, porte de manière étanche, au moins par une surface annulaire plane située du côté plan tourné à l'opposé des nervures annulaires (14, 15), contre une surface plane de contact de ladite face intérieure dudit couvercle (8) du conteneur.

6. Conteneur stérile selon la revendication 2, **caractérisé par le fait que** la platine d'embase (16) est pourvue, dans une région radiale interposée entre le filetage et la nervure annulaire située le plus à l'intérieur, d'orifices (13') de communication avec les orifices de ventilation (13) du couvercle (8) dudit conteneur, pareillement disposés dans cette région radiale.

7. Conteneur stérile selon la revendication 1 ou 2, **caractérisé par le fait qu'**une coiffe discoïdale protectrice (23), ménagée à la face extérieure du couvercle (8) dudit conteneur ou mise en place sur cette dernière, occulte les orifices de ventilation dudit couvercle avec espacement axial et est munie, dans un anneau marginal périphérique (25), d'au moins un orifice radial de passage (26) présentant un espacement axial minimal (a') vis-à-vis de la surface extérieure (27) dudit couvercle (8) du conteneur qui reçoit ledit anneau marginal (25).

8. Conteneur stérile selon la revendication 7, **caractérisé par le fait qu'**un anneau marginal (25) d'une coiffe protectrice (23), mise en place sur la face extérieure du couvercle (8) dudit conteneur, ceinture une cavité aplatie (29) en communication avec les orifices de ventilation (13) dudit couvercle (8), et porte de manière étanche, par une surface annulaire plane (30), contre la surface extérieure (27) dudit couvercle (8) du conteneur.

9. Conteneur stérile selon la revendication 7, **caractérisé par le fait que** l'orifice radial de passage de l'anneau marginal (25) se présente comme un évidement (26) du type fente, s'étendant parallèlement à sa surface annulaire plane.

10. Conteneur stérile selon la revendication 8 ou 9, **caractérisé par le fait que** l'anneau marginal (25) est muni de plusieurs orifices de passage (26) du type fentes, agencés avec répartition dans la direction périphérique.

11. Conteneur stérile selon la revendication 3 ou 4, **caractérisé par le fait que** la platine d'embase (16/1) est fixée amoviblement au couvercle (8) dudit conteneur au moyen d'un tenon fileté (21) qui traverse un perçage (36/1) dudit couvercle (8) du conteneur et est en prise avec un filetage complémentaire central (18) de ladite platine d'embase (16/1), ou de la platine de recouvrement (17/1).

12. Conteneur stérile selon les revendications 3 et 9, **caractérisé par le fait que** le tenon fileté (21') assure également la fixation de la coiffe protectrice (23) sur la face extérieure du couvercle (8) dudit conteneur.

13. Conteneur stérile selon la revendication 12, **caractérisé par le fait que** le tenon fileté (21') fait partie intégrante de la coiffe protectrice (23).

14. Conteneur stérile selon la revendication 1, 2 ou 3, **caractérisé par le fait que** la platine d'embase (16/1) est centrée au moyen d'un anneau marginal (36) pénétrant dans une rainure annulaire adaptée (38) du couvercle (8) dudit conteneur, concentrique à la région marginale des orifices de ventilation (13).

15. Conteneur stérile selon la revendication 14, **caractérisé par le fait que** l'étanchéité réciproque de l'anneau marginal (36) et de la rainure annulaire (38) est assurée à l'aide d'un moyen d'étanchement, en particulier au moyen d'une bague torique élastique ou d'une garniture d'étanchement au silicone.
